(19) Europäisches Patentamt
European Patent Office
Office européen des brevets

(11) Veröffentlichungsnummer: **0 461 501 A1**

# (12) EUROPÄISCHE PATENTANMELDUNG

(21) Anmeldenummer: **91108986.0**

(51) Int. Cl.5: **C07D 215/56**, A61K 31/47

(22) Anmeldetag: **01.06.91**

(30) Priorität: **14.06.90 DE 4019023**

(43) Veröffentlichungstag der Anmeldung:
**18.12.91 Patentblatt 91/51**

(84) Benannte Vertragsstaaten:
**AT BE CH DE DK ES FR GB GR IT LI LU NL SE**

(71) Anmelder: **BAYER AG**
**W-5090 Leverkusen 1 Bayerwerk(DE)**

(72) Erfinder: **Preiss, Michael, Dr.**
**Paul-Ehrlich-Strasse 12**
**W-5600 Wuppertal 1(DE)**

## (54) Verfahren zur Herstellung von Chinolincarbonsäuren.

(57) Gegenstand der Erfindung ist ein Verfahren zur Herstellung von Chinolincarbonsäuren, bei dem die Bildung von Nebenprodukten stark reduziert ist.

EP 0 461 501 A1

Gegenstand der Erfindung ist ein Verfahren zur Herstellung von Chinolincarbonsäuren, das dadurch gekennzeichnet ist, daß Verbindungen der Formel (I)

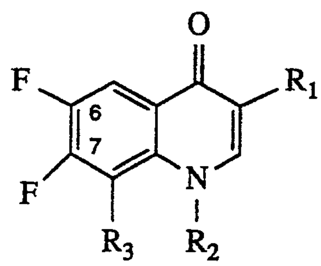

(I)

worin

| | |
|---|---|
| $R_1$ | COO-Alkyl, Cyano, $CONAlk_2$, COOH |
| $R_2$ | substituiertes oder unsubstituiertes Alkyl, substituiertes oder unsubstituiertes Cycloalkyl, NH-Alkyl, $NAlk_2$, Aryl, fluorsubstituiertes Aryl, |
| $R_2$, $R_3$ | miteinander verbunden sein können durch eine substituierte oder unsubstituierte Alkylkette, die von Heteroatomen unterbrochen sein kann, |
| $R_3$ | Alkyl, Cycloalkyl, Halogen, Cyano, Nitro, Alkylthio, Alkoxy, Carboxy, Wasserstoff |

sein können, mit Verbindungen der Formel (II)

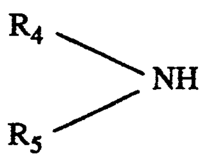

(II)

worin

| | |
|---|---|
| $R_4$ und $R_5$ | gleich oder verschieden sind und für Wasserstoff, einen verzweigten oder unverzweigten Alkyl-, Alkenyl- oder Alkinylrest mit 1 bis 12 Kohlenstoffatomen stehen, der gegebenenfalls durch Hydroxylgruppen, Alkoxy-, Alkylmercapto- oder Dialkyl-amino-gruppen mit 1 bis 3 Kohlenstoffatomen in jedem Alkylrest, die Nitril- sowie eine Alkoxycarbonyl-Gruppe mit 1 bis 4 Kohlenstoffatomen im Alkoholteil substituiert sein kann, ferner Cycloalkyl mit 3 bis 6 Kohlenstoffatomen bedeuten und weiterhin gemeinsam mit dem Stickstoffatom, das sie substituieren und gegebenenfalls einem Heteroatom wie z.B. Sauerstoff, Schwefel oder $NR_6$ einen 3 bis 7-gliedrigen Ring bilden, der ein- oder mehrfach durch Alkyl- oder Alkenylgruppen mit 1 bis 6 Kohlenstoffatomen, Hydroxyl-, Alkoxy- und Alkylmercaptogruppen mit 1 bis 3 Kohlenstoffatomen, eine Alkoxycarbonylgruppe mit 1 bis 4 Kohlenstoffatomen im Alkoholteil, die Nitrilgruppe sowie einen Phenylrest substituiert sein kann und ferner eine Doppelbindung enthalten kann und |
| $R_6$ | Wasserstoff, eine verzweigte oder unverzweigte Alkyl-, Alkenyl- oder Alkinylgruppe mit 1 bis 6 Kohlenstoffatomen, die gegebenenfalls durch Hydroxyl, Alkoxy-, Alkylmercapto- und Dialkylaminogruppen mit 1 bis 3 Kohlenstoffatomen, einen Alkylrest, die Alkoxycarbonylgruppe mit 1 bis 4 Kohlenstoffatomen im Alkoholteil substituiert sein kann, eine gegebenenfalls im Phenylrest substituierte Phenylalkylgruppe mit bis zu 4 Kohlenstoffatomen im aliphatischen Teil, sowie eine gegebenenfalls substituierte Phenyl- oder Naphthylgruppe oder ein heterocyclischer Rest wie beispielsweise einen Pyridin-, Pyrimidin-, Thiazol-, oder Benzthiazolkern darstellt, ferner eine gegebenenfalls durch einen Phenylrest substituierte Alkoxycarbonylgruppe mit 1 bis 4 Kohlenstoffatomen im Alkoholteil, ein Alkanoylrest mit 1 bis 6 Kohlenstoffatomen, ein Benzoylrest, ein gegebenenfalls substituierter $C_1$-$C_6$-Alkyl- oder Phenylsulfonylrest sowie ein gegebenenfalls substituierter Aminosulfonylrest bedeutet, wobei als Substituenten von Phenyl- und Naphthylgruppen Halogen, Alkyl-, Alkoxy- oder Alkylenmercaptogruppen mit 1 bis 3 Kohlenstoffatomen, Phenyloxy-, Phenylmercapto-, Trifluormethyl-, Nitro-, Nitril- oder Carbonestergruppen mit 1 bis 4 Kohlenstoffatomen im Alkoholteil in Frage kommen, |

in Gegenwart von Wasser oder Gemischen aus Wasser und wasserlöslichen Lösungsmitteln bei Temperaturen von 95 bis 120° C gegebenenfalls unter Druck, zu Verbindungen der Formel (III) umgesetzt werden.

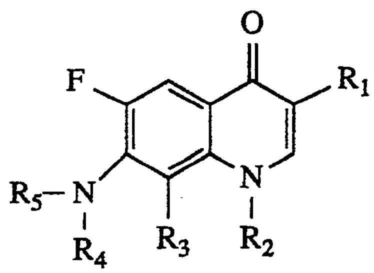

(III)

Bevorzugt wird das Verfahren zur Herstellung von Verbindungen angewendet, bei denen in Formel (I)

$R_1$ COO-Alkyl, COOH,

$R_2$ substituiertes oder unsubstituiertes Alkyl, substituiertes oder unsubstituiertes Cycloalkyl, fluorsubstituiertes Aryl,

$R_3$ Halogen, Nitro, Alkylthio, Alkoxy, Wasserstoff, sind und in Formel (II)

$R_4$ und $R_5$ gemeinsam mit dem Stickstoffatom, das sie substituieren und gegebenenfalls einem Heteroatom wie z.B. Sauerstoff, Schwefel oder $NR^6$ einen 3 bis 7-gliedrigen Ring bilden, der ein- oder mehrfach durch Alkyl- oder Alkenylgruppen mit 1 bis 6 Kohlenstoffatomen, Hydroxyl-, Alkoxy- und Alkylmercaptogruppen mit 1 bis 3 Kohlenstoffatomen, eine Alkoxycarbonylgruppe mit 1 bis 4 Kohlenstoffatomen im Alkoholteil, die Nitrilgruppe sowie einen Phenylrest substituiert sein kann und ferner eine Doppelbindung enthalten kann und

$R_6$ Wasserstoff, eine verzweigte oder unverzweigte Alkyl-, Alkenyl- oder Alkinylgruppe mit 1 bis 6 Kohlenstoffatomen, die gegebenenfalls durch Hydroxyl, Alkoxy-, Alkylmercapto- und Dialkylaminogruppen mit 1 bis 3 Kohlenstoffatomen, einen Alkylrest, die Alkoxycarbonylgruppe mit 1 bis 4 Kohlenstoffatomen im Alkoholteil substituiert sein kann, bedeutet.

Besonders bevorzugt wird das Verfahren zur Herstellung von Verbindungen verwendet, bei denen in Formel (I)

$R_1$ COO-Alkyl, COOH,

$R_2$ substituiertes oder unsubstituiertes Cycloalkyl,

$R_3$ Halogen oder Wasserstoff, sind und in Formel (II)

$R_4$ und $R_5$ gemeinsam mit dem Stickstoffatom, das sie substituieren und gegebenenfalls einem Heteroatom $NR^7$ einen 5- oder 6-gliedrigen Ring bilden, der ein- oder mehrfach durch Alkyl- oder Alkenylgruppen mit 1 bis 6 Kohlenstoffatomen, Hydroxyl-, Alkoxy- und Alkylmercaptogruppen mit 1 bis 3 Kohlenstoffatomen substituiert sein kann und ferner eine Doppelbindung enthalten kann und

$R_6$ Wasserstoff, eine verzweigte oder unverzweigte Alkyl-, Alkenyl- oder Alkinylgruppe mit 1 bis 6 Kohlenstoffatomen, die gegebenenfalls durch Hydroxyl, Alkoxy-, Alkylmercapto- und Dialkylaminogruppen mit 1 bis 3 Kohlenstoffatomen, einen Alkylrest, die Alkoxycarbonylgruppe mit 1 bis 4 Kohlenstoffatomen im Alkoholteil substituiert sein kann, bedeutet.

Es ist als ausgesprochen überraschend zu bezeichnen, daß die Substitution des Fluoratoms im Gegensatz zu z.B. Chlor und Brom an der gleichen Stelle (Position 7, Formel (I)) mit ca. 100 mal höherer Geschwindigkeit abläuft. Dies ist aus den Abbildungen 1 und 2 ersichtlich. Daraus ergibt sich, daß die Reaktion schon bei Temperaturen um 100° C, z.B. in Wasser, mit ausreichender Geschwindigkeit abläuft.

Aus der Möglichkeit, bei Temperaturen um 100° C zu arbeiten, ergeben sich eine Reihe von Vorteilen:

a) Es ist dadurch möglich, in Wasser oder wäßrigen Gemischen zu arbeiten, was kostengünstig und ökologisch vorteilhaft ist.

b) Bei der Darstellung von mit Diaminen substituierten Verbindungen (III) wird die Bildung von dimeren, oligomeren und polymeren Nebenprodukten stark zurückgedrängt, da die Temperatur für eine Kondensation gemäß nachstehendem Reaktionsschema nicht hoch genug ist.

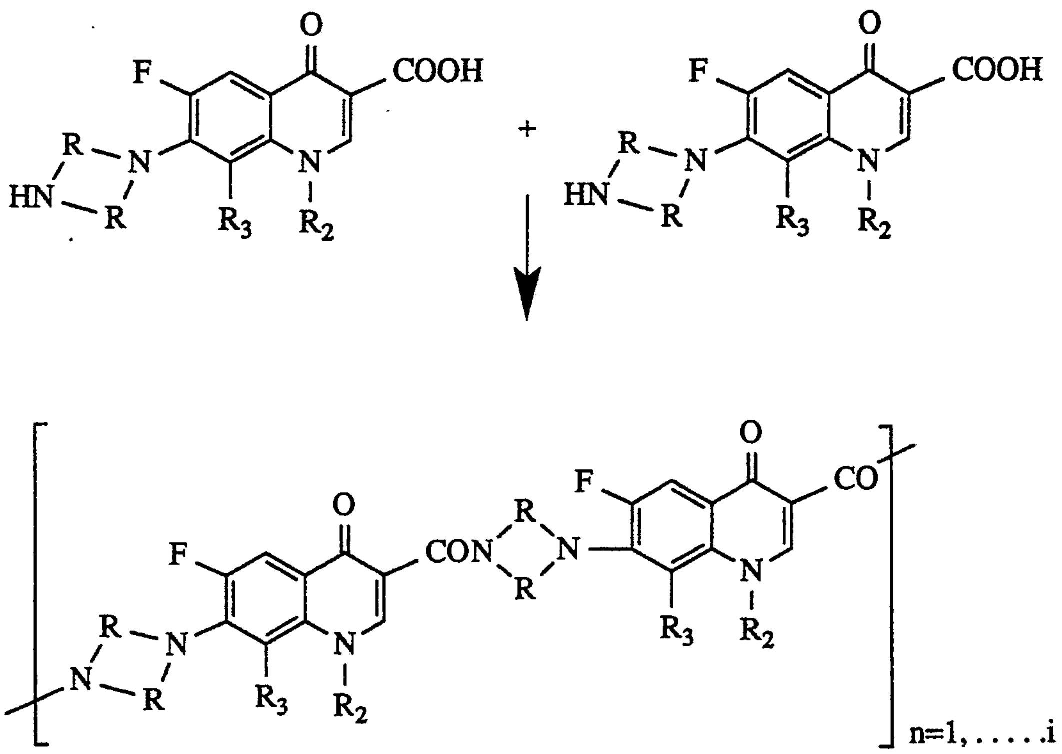

c) Die Bildung eines weiteren Nebenproduktes wird unterdrückt. Die decarboxylierten Verbindungen (VIII) (im Falle von $R_1$ = COOH) treten nicht mehr auf.

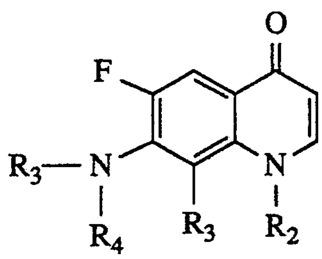

(VIII)

Bei den üblicherweise angewendeten Temperaturen über 140 °C können 5 Mol-% und mehr dieser Produkte (VIII) gebildet werden, die in einem zusätzlichen Verfahrensschritt abgetrennt werden müssen.

Ein weiterer überraschender Befund ist die extrem hohe Selektivität der Fluor-Substitution. Es wird praktisch ausschließlich das Fluoratom in Position 7 der Verbindung (I) ausgetauscht. Dies manifestiert sich in Ausbeuten an reinem (III) um 95 % der Theorie.

Aus der Kombination der genannten Vorteile, Durchführung der Reaktion in Wasser oder Wassergemischen, der hohen Selektivität, und dem minimalen Anteil an Nebenprodukten ergibt sich ein weiterer Vorteil: bisher mußte die Betainform von (III) isoliert werden, um die unerwünschten Nebenprodukte (ca. 20 %) zu entfernen. Da letztere praktisch nicht mehr vorhanden sind, kann auf die Isolierung der Betainform verzichtet werden, zumal die nachfolgende Umsetzung zum pharmazeutisch anwendbaren Salz von (III) in Wasser oder Wassergemischen erfolgt.

Die Umsetzung von Verbindungen des Typs (IV) mit Aminen wie (II) ist prinzipiell aus der EP-A-0 078 362 bekannt. Die Selektivität der Substitution des $C_7$-Substituenten (Chlor) beträgt etwa 10:1 in Konkurrenz mit der $C_6$-Substitution (Fluor); d.h. 1/10 des wertvollen Ausgangsmaterials (IV) geht für die nicht verwertbaren Verbindungen (VI) verloren.

(IV) 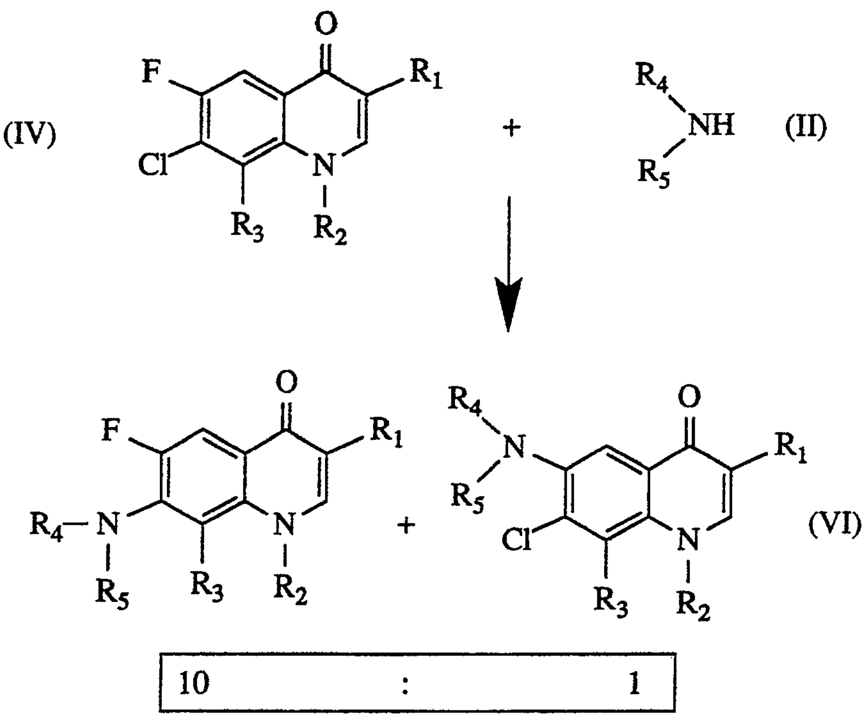 + (II)


(III) + (VI)

Desweiteren ist nachteilig, daß durchweg bei Temperaturen um 140° C und zudem in einem organischen Lösungsmittel gearbeitet wird.

Ausgangsverbindungen der Formel (I) sind aus der EP-A-0 169 993 und EP-A-0 274 033 bekannt; es wird jedoch auch hier bei Temperaturen von 140° C in einem organischen Lösungsmittel gearbeitet, wodurch man mit den oben beschriebenen Nebenprodukten rechnen muß.

Die Bildung der Verbindungen (VI) wirkt sich in zweifacher Weise nachteilig aus: zum einen geht, wie oben erwähnt, 1/10 des Ausgangsmaterials verloren, zum anderen ist die Abtrennung der Verbindung (VI), da ihre physikalischen Eigenschaften sehr ähnlich (III) sind, sehr schwierig, so daß immer ein gewisser Anteil an (III) in der unerwünschten Verbindung (VI) verbleibt. Dieser Verlust beläuft sich auf über 10 %. Man erhält also ca. 20 % weniger an (III), wenn als Ausgangsmaterialien Verbindungen des Typs (VII) mit X ≠ F eingesetzt werden.

(VII) 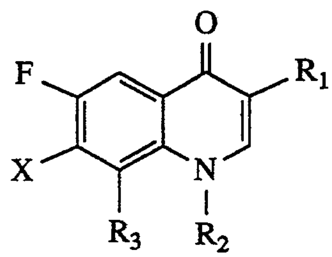

Die Vorteile des erfindungsgemäßen Verfahrens sind demnach:

- über 20 % höhere Ausbeuten
- praktisch keine Nebenprodukte
- Wasser als Lösungsmittel
- niedrigere Reaktionstemperatur
- Zeitersparnis durch Wegfall einer Verfahrensstufe.

Das Verhältnis der Reaktionspartner (I) zu (II) beträgt 1:1 bis 1:10, vorzugsweise 1:2 bis 1:4. Die Reaktion wird vorzugsweise in Wasser bei der sich einstellenden Rückflußtemperatur durchgeführt. Es können aber auch alle anderen, unter den angewandten Reaktionsbedingungen inerten, mit Wasser mischbaren Lösungsmittel anteilig eingesetzt werden. Gegebenenfalls kann auch bei Temperaturen, die über der Rückflußtemperatur liegen unter Druck gearbeitet werden. Ein Teil des Amins (II) kann auch durch andere Basen, z.B. Alkalilaugen oder tertiäre Amine ersetzt werden.

Beispiele

Beispiel 1

In eine zum Rückfluß erhitzte Lösung aus 26,7 Gew.-Teilen Piperazin in 200 Vol-Teilen Wasser werden 26,5 Gew.-Teile 1-Cyclopropyl-6,7-difluor-1,4-dihydro-4-oxo-3-chinolincarbonsäure eingetragen. Es wird 90 Minuten am Rückfluß gehalten und dann abgekühlt. Der Niederschlag wird abgetrennt, mit Wasser gewaschen und getrocknet. Man erhält 31,5 Gew.-Teile (95,2 % der Theorie). 1-Cyclopropyl-6-fluor-1,4-dihydro-4-oxo-7-(1-piperazinyl)-3-chinolincarbonsäure.

Beispiel 2

26,7 Gew.-Teile Piperazin und 26,5 Gew.-Teile 1-Cyclopropyl-6,7-difluor-1,4-dihydro-4-oxo-3-chinolincarbonsäure werden in 200 Vol-Teilen Wasser 60 Minuten im Autoklaven auf 120 °C erhitzt und dann wie in Beispiel 1 aufgearbeitet. Man erhält 30,9 Gew.-Teile (93,4 % der Theorie) 1-Cyclopropyl-6-fluor-1,4-dihydro-4-oxo-7-(1-piperazinyl)-3-chinolincarbonsäure.

Beispiel 3

17,2 Gew.-Teile Piperazin, 4 Gew.-Teile Natriumhydroxid und 26,5 Gew.-Teile 1-Cyclopropyl-6,7-difluor-1,4-dihydro-4-oxo-3-chinolincarbonsäure in 200 Vol-Teilen Wasser werden 150 Minuten am Rückfluß erhitzt und dann wie in Beispiel 1 aufgearbeitet. Man erhält 29,8 Gew.-Teile (90,0 % der Theorie) 1-Cyclopropyl-6-fluor-1,4-dihydro-4-oxo-7-(1-piperazinyl)-3-chinolincarbonsäure.

Beispiel 4

26,7 Gew.-Teile Piperazin und 26,5 Gew.-Teile 1-Cyclopropyl-6,7-difluor-1,4-dihydro-4-oxo-3-chinolincarbonsäure werden in 200 Vol-Teilen Wasser 90 Minuten am Rückfluß erhitzt. Dann wird auf 78 °C abgekühlt, 200 Vol-Teile Ethanol und soviel Salzsäure zugegeben bis das Produkt gelöst ist. Es wird von einer geringen Menge Unlöslichem abgetrennt und abgekühlt. Der Niederschlag wird abgesaugt, mit Ethanol gewaschen und getrocknet. Man erhält 34,7 Gew.-Teile (89,9 %) 1-Cyclopropyl-6-fluor-1,4-dihydro-4-oxo-7-(1-piperazinyl)-4-chinolincarbonsäure-Hydrochlorid-Monohydrat.

Beispiel 5

26,5 Gew.-Teile 1-Cyclopropyl-6,7-difluor-1,4-dihydro-4-oxo-3-chinolin-carbonsäure und 35,3 Gew.-Teile 1-Ethyl-piperazin werden in 200 Vol-Teilen Wasser 90 Minuten am Rückfluß erhitzt, abgekühlt, mit Säure auf pH 7,5 gebracht und aufgearbeitet. Man erhält 34,0 g (94,7 %) 1-Cyclopropyl-6-fluor-1,4-dihydro-4-oxo-7-(4-ethyl-1-piperazinyl)-3-chinolincarbonsäure.

**Patentansprüche**

**1.** Verfahren zur Herstellung von Chinolincarbonsäuren, dadurch gekennzeichnet, daß Verbindungen der Formel (I)

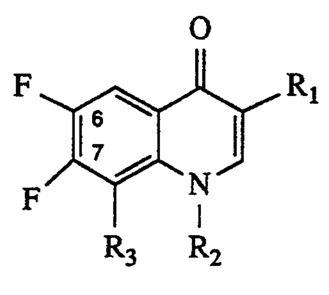

(I)

worin

$R_1$ COO-Alkyl, Cyano, $CONAlk_2$, COOH

$R_2$ substituiertes oder unsubstituiertes Alkyl, substituiertes oder unsubstituiertes Cycloalkyl, NH-Alkyl, $NAlk_2$, Aryl, fluorsubstituiertes Aryl,

$R_2$, $R_3$ miteinander verbunden sein können durch eine substituierte oder unsubstituierte Alkylkette, die von Heteroatomen unterbrochen sein kann,

$R_3$ Alkyl, Cycloalkyl, Halogen, Cyano, Nitro, Alkylthio, Alkoxy, Carboxy, Wasserstoff

sein können, mit Verbindungen der Formel (II)

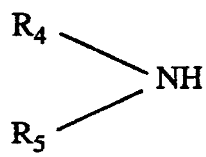

(II)

worin

$R_4$ und $R_5$ gleich oder verschieden sind und für Wasserstoff, einen verzweigten oder unverzweigten Alkyl-, Alkenyl- oder Alkinylrest mit 1 bis 12 Kohlenstoffatomen stehen, der gegebenenfalls durch Hydroxylgruppen, Alkoxy-, Alkylmercapto- oder Dialkyl-aminogruppen mit 1 bis 3 Kohlenstoffatomen in jedem Alkylrest, die Nitril- sowie eine Alkoxycarbonyl-Gruppe mit 1 bis 4 Kohlenstoffatomen im Alkoholteil substituiert sein kann, ferner Cycloalkyl mit 3 bis 6 Kohlenstoffatomen bedeutet und weiterhin gemeinsam mit dem Stickstoffatom, das sie substituieren und gegebenenfalls einem Heteroatom wie z.B. Sauerstoff, Schwefel oder $NR^6$ einen 3 bis 7-gliedrigen Ring bilden, der ein- oder mehrfach durch Alkyl- oder Alkenylgruppen mit 1 bis 6 Kohlenstoffatomen, Hydroxyl-, Alkoxy- und Alkylmercaptogruppen mit 1 bis 3 Kohlenstoffatomen, eine Alkoxycarbonylgruppe mit 1 bis 4 Kohlenstoffatomen im Alkoholteil, die Nitrilgruppe sowie einen Phenylrest substituiert sein kann und ferner eine Doppelbindung enthalten kann und

$R_6$ Wasserstoff, eine verzweigte oder unverzweigte Alkyl-, Alkenyl- oder Alkinylgruppe mit 1 bis 6 Kohlenstoffatomen, die gegebenenfalls durch Hydroxyl, Alkoxy-, Alkylmercapto- und Dialkylaminogruppen mit 1 bis 3 Kohlenstoffatomen, einen Alkylrest, die Alkoxycarbonylgruppe mit 1 bis 4 Kohlenstoffatomen im Alkoholteil substituiert sein kann, eine gegebenenfalls im Phenylrest substituierte Phenylalkylgruppe mit bis zu 4 Kohlenstoffatomen im aliphatischen Teil, sowie eine gegebenenfalls substituierte Phenyl- oder Naphthylgruppe oder ein heterocyclischer Rest wie beispielsweise einen Pyridin-, Pyrimidin-, Thiazol-, oder Benzthiazolkern darstellt, ferner eine gegebenenfalls durch einen Phenylrest substituierte Alkoxycarbonylgruppe mit 1 bis 4 Kohlenstoffatomen im Alkoholteil, ein Alkanoylrest mit 1 bis 6 Kohlenstoffatomen, ein Benzoylrest, ein gegebenenfalls substituierter $C_1$-$C_6$-Alkyl- oder Phenylsulfonylrest sowie ein gegebenenfalls substituierter Aminosulfonylrest bedeutet, wobei als Substituenten von Phenyl- und Naphthylgruppen Halogen, Alkyl-, Alkoxy- oder Alkylenmercaptogruppen mit 1 bis 3 Kohlenstoffatomen, Phenyloxy-, Phenylmercapto-, Trifluormethyl-, Nitro-, Nitril- oder Carbonestergruppen mit 1 bis 4 Kohlenstoffatomen im Alkoholteil in Frage kommen,

in Gegenwart von Wasser oder Gemischen aus Wasser und wasserlöslichen Lösungsmitteln bei Temperaturen von 95 bis 120 °C gegebenenfalls unter Druck, zu Verbindungen der Formel (III) umgesetzt werden.

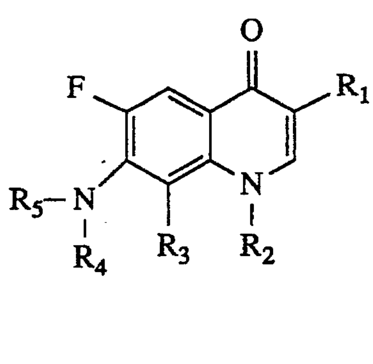

(III)

2. Verfahren gemäß Anspruch 1, dadurch gekennzeichnet, daß in Formel (I)
   $R_1$ COO-Alkyl, COOH,
   $R_2$ substituiertes oder unsubstituiertes Alkyl, substituiertes oder unsubstituiertes Cycloalkyl, fluorsubstituiertes Aryl,
   $R_3$ Halogen, Nitro, Alkylthio, Alkoxy, Wasserstoff,
   sein können,

   und in Formel (II)
   $R_4$ und $R_5$ gemeinsam mit dem Stickstoffatom, das sie substituieren und gegebenenfalls einem Heteroatom wie z.B. Sauerstoff, Schwefel oder $NR^6$ einen 3 bis 7-gliedrigen Ring bilden, der ein- oder mehrfach durch Alkyl- oder Alkenylgruppen mit 1 bis 6 Kohlenstoffatomen, Hydroxyl-, Alkoxy- und Alkylmercaptogruppen mit 1 bis 3 Kohlenstoffatomen, eine Alkoxycarbonylgruppe mit 1 bis 4 Kohlenstoffatomen im Alkoholteil, die Nitrilgruppe sowie einen Phenylrest substituiert sein kann und ferner eine Doppelbindung enthalten kann und
   $R_6$ Wasserstoff, eine verzweigte oder unverzweigte Alkyl-, Alkenyl- oder Alkinylgruppe mit 1 bis 6 Kohlenstoffatomen, die gegebenenfalls durch Hydroxyl, Alkoxy-, Alkylmercapto- und Dialkylaminogruppen mit 1 bis 3 Kohlenstoffatomen, einen Alkylrest, die Alkoxycarbonylgruppe mit 1 bis 4 Kohlenstoffatomen im Alkoholteil substituiert sein kann, bedeutet.

3. Verfahren gemäß Anspruch 1, dadurch gekennzeichnet, daß in Formel (I)
   $R_1$ COO-Alkyl, COOH,
   $R_2$ substituiertes oder unsubstituiertes Cycloalkyl,
   $R_3$ Halogen oder Wasserstoff,
   sein können,

   und in Formel (II)
   $R_4$ und $R_5$ gemeinsam mit dem Stickstoffatom, das sie substituieren und gegebenenfalls einem Heteroatom $NR_6$ einen 5- oder 6-gliedrigen Ring bilden, der ein- oder mehrfach durch Alkyl- oder Alkenylgruppen mit 1 bis 6 Kohlenstoffatomen, Hydroxyl-, Alkoxy- und Alkylmercaptogruppen mit 1 bis 3 Kohlenstoffatomen substituiert sein kann und ferner eine Doppelbindung enthalten kann und
   $R_6$ Wasserstoff, eine verzweigte oder unverzweigte Alkyl-, Alkenyl- oder Alkinylgruppe mit 1 bis 6 Kohlenstoffatomen, die gegebenenfalls durch Hydroxyl, Alkoxy-, Alkylmercapto- und Dialkylaminogruppen mit 1 bis 3 Kohlenstoffatomen, einen Alkylrest, die Alkoxycarbonylgruppe mit 1 bis 4 Kohlenstoffatomen im Alkoholteil substituiert sein kann, bedeutet.

Europäisches Patentamt

# EUROPÄISCHER RECHERCHENBERICHT

Nummer der Anmeldung

EP 91 10 8986

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 140 116 (HOKURIKA PHARM.)<br>* Seite 5; Tabelle 4, Beispiele 10,12,15 * | 1-3 | C 07 D 215/56<br>A 61 K 31/47 |
| X | EP-A-0 247 464 (FUJISAWA PHARM.)<br>* Seite 10, Zeilen 4-9; Beispiel 1 * | 1 | |
| A | EP-A-0 264 050 (BAYER AG)<br>* Seite 5, Zeilen 36-40 * | 1-3 | |
| A | EP-A-0 178 388 (KYORIN PHARM.)<br>* Seite 2 * | 1-3 | |
| A | EP-A-0 226 961 (WARNER-LAMBERT)<br>* Seite 3, Zeilen 28-32 * | 1-3 | |

RECHERCHIERTE SACHGEBIETE (Int. Cl.5)

C 07 D 215/00
A 61 K 31/00

Der vorliegende Recherchenbericht wurde für alle Patentansprüche erstellt

| Recherchenort | Abschlußdatum der Recherche | Prüfer |
|---|---|---|
| Den Haag | 16 September 91 | DE JONG B.S. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument